# EUROPEAN PATENT SPECIFICATION

(11) **EP 1 448 774 B1**
(45) Date of publication and mention of the grant of the patent: **07.06.2006**
(21) Application number: 02779700.0
(22) Date of filing: 20.11.2002
(51) Int. Cl.: C12N 15/10, C12N 1/06

(54) **EXTRACTION OF NUCLEIC ACID**
NUKLEINSÄURE-EXTRAKTION
EXTRACTION D'ACIDE NUCLEIQUE

(30) Priority: 20.11.2001 GB 0127809
(43) Date of publication of application: 25.08.2004
(73) Proprietor: INVITROGEN CORPORATION, Carlsbad, CA 92008 (US)
(72) Inventor: BAKER, Matthew, Maidstone, Kent ME15 9UJ (GB); TAYLOR, Matthew, Sittingbourne, Kent ME10 2JT (GB); UPPAL, Shilpa, Canterbury, Kent CT3 1AU (GB)
(74) Representative: Kiddle, Simon John
(86) International application number: PCT/GB2002/005209
(87) International publication number: WO 2003/046177

(56) References cited:
- EP-A- 0 657 530
- MERK S ET AL: "Comparison of different methods for the isolation of Burkholderia cepacia DNA from pure cultures and waste water." INTERNATIONAL JOURNAL OF HYGIENE AND ENVIRONMENTAL HEALTH. GERMANY NOV 2001, vol. 204, no. 2-3, November 2001 (2001-11), pages 127-131, XP009005400 ISSN: 1438-4639

## Description

### Field of the Invention

The present invention relates to nucleic acid purification, and in particular to a method of obtaining a sample of target nucleic acid from bacterial cells containing the target nucleic acid and genomic DNA or RNA.

### Background of the Invention

Current methods of purifying non-genomic nucleic acids, such as vector DNA or RNA, rely on extensive cell lysis or cell wall degradation to release the nucleic acid present in the cells, using cell lysing enzymes such as lysozyme, chaotropic agents or extremes of pH and heat (see for example the methods disclosed in Maniatis, 'Molecular Cloning, A Laboratory Manual', Book 1, Sections 1.21-1.23, Cold Spring Harbor Press, 1989). However, while this approach releases the non-genomic nucleic acid from the cells, it is accompanied by the majority of the other host genomic nucleic acid contained in the cell, and other impurities such as cellular endotoxins. This necessitates a sequence of difficult and time consuming purification steps in order purify target nucleic acid molecules from these impurities. These steps are needed as the release of RNA or host chromosomal DNA can then interfere with the downstream analysis or functionality of the target nucleic acid which, if present in the sample of the target nucleic acid. Further, extensive cell lysis often results in the generation of a viscous mass of cellular material making further purification difficult.

Calvin and Hanawalt (J. Bacteriol, 170(6): 2796-2801, 1988) discloses the use of reverse electroporation for the recovery of plasmid DNA from *E. coli.* While plasmids were recovered in the method, most of the conditions tested also led to the extraction of cellular DNA and RNA. In order to obtain the plasmids, electrical field strengths of 13,000-15,000 V/cm were required.

WO 00/29563 (Cambridge Molecular Technologies Limited) discloses a method of separating plasmid from genomic DNA employing a water immiscible organic solvent, a chaotrope and heating to at least 65°C. This led to the extraction of the plasmid DNA into the organic phase.

EP 0 657 530 A (Gen-Probe Inc) discloses a method of extracting nucleic acid from bacterial cells employing a permeabilising reagent comprising a non-ionic detergent and a metal chelating agent, such as EDTA, in combination with heating to 80-100°C.

An additional problem is that the procedures to purify a sample of the target nucleic acid from the impurities typically requires several,centrifugation steps and during the purification procedures, the volume of the sample changes significantly. These factors mean that existing purification protocols need to be carried out by a technician and are not easily automated.

### Summary of the Invention

Broadly, the present invention provides method of obtaining a sample of target nucleic acid from bacterial cells containing the target nucleic acid and genomic DNA or RNA. In contrast to prior art protocols, this method does not require the cells containing the target nucleic acid to be lysed and instead relies on the observation that after separation from culture broth or medium, cells can be resuspended in an aqueous medium and the target nucleic acid released into the aqueous medium through the cell walls without the need.for cell lysis. The present invention may in preferred embodiments avoid the heating used in many prior art nucleic acid extraction methods. The present invention is particularly applicable to the separation of non-genomic nucleic acid, such as cellular vector DNA or RNA, self-replicating satellite nucleic acids or plasmid DNA, from genomic nucleic acids, such as host cell chromosomes and ribosomal RNA.

Accordingly, in a first aspect, the present invention provides a method of obtaining a sample of target nucleic acid from bacterial cells containing the target nucleic acid and genomic nucleic acid, the method comprising the steps of:
separating the cells from culture broth;
suspending the cells in an aqueous medium which causes the target nucleic acid to leak from the cells into the aqueous medium, wherein the aqueous medium is water, a law salt buffer, a salt solution, a divalent or trivalent metal salt solution or a sugar solution; and
obtaining the sample of the nucleic acid from the aqueous medium;
wherein the cells are substantially not lysed during the above steps and substantially retain the genomic nucleic acid within the cells and wherein the method is carried out at a temperature of less than 60°C and in the absence of an electrical field capable of causing cell poration. Preferably, the method does not substantially cause the release of cellular endotoxins, thereby allowing the separation of the target nucleic acid from the cellular endotoxins, in addition to genomic nucleic acid or RNA.

While not wishing to be bound by any particular theory, the inventors believe that the change in the environment of the cells from the conditions in culture broth to the conditions in the aqueous medium, typically at lower ionic strength, makes the cell walls 'leaky' or slightly porous to the target nucleic acid, and especially nucleic acid such as vectors. This allows the target nucleic acid to diffuse into the aqueous medium without the need to lyse the cells, e.g. by contacting the cells with lysozyme, chaotropes or extremes of pH or heat, with the result that the cells retain impurities such as genomic DNA and cellular proteins, thereby avoiding the need for complicated procedures to remove these materials from a sample comprising the impurities and the target nucleic acid. In a preferred embodiment of the invention, the target nucleic acids may be 100kb or less, or more preferably 50kb or less, or more preferably 20kb or less or even more preferably 10kb or less in size. The size of nucleic acids can be determined by those skilled in the art, e.g. using gel electrophoresis technique employing a polyacrylamide or agarose gel, e.g. see Ausubel et al, 'Short Protocols in Molecular Biology', John Wiley and Sons, NY, 1992.

In the present invention, "not substantially lysed" means preferably less than 20%, more preferably less than 10%, more preferably less than 5%, more preferably less than 2% and most preferably less than 1% of the cells in the population treated according to the method are lysed.
The extent of cell lysis can readily be determined, e.g. by counting lysed and non-lysed cells present in a sample under a microscope.

In the method, the target nucleic acid is non-genomic nucleic acid which is separated from genomic nucleic acid retained inside the cells. Non-genomic nucleic acid includes vectors, plasmids, self replicating satellite nucleic acid or cosmid DNA, or vector RNA. Other forms of target nucleic acids may include bacteriophages such as Lambda, M13 and viral nucleic acids. In a preferred embodiment, the non-genomic nucleic acid sample is plasmid DNA.

The method is widely applicable to many different types of bacterial host cells. For example Gram negative and Gram positive bacteria and filamentous bacteria. The use of gram negative bacteria is preferred, such as *E. Coli* strains, e.g. strains JM109 or HB101.

Typically, the cells are treated at ambient temperatures between 0 and 40°C. Unlike many of the prior art methods, the present invention employs temperatures less than about 60°C, and more preferably less than about 40°C. Preferred temperature ranges employed in the method of the invention are between 0 and 60°C, between 0 and 40°C and most preferably between 15 and 40°C.

The present invention includes the use of a variety of different conditions to cause the release of target nucleic acid from the cells into the surrounding aqueous medium. Examples of preferred aqueous media include water, salts such as NaCl, in hypotonic or hypertonic conditions. Examples of low salt buffers include Tris. HCl, e.g. at pH 6-9, optionally including EDTA, a potassium salt such as potassium acetate, optionally including KCl, or a divalent or trivalent metal salt such as CaCl₂, preferably at a concentration between 10 and 250mM, and more preferably between 50 and 150mM.

Alternatively or additionally, a sugar solution can also be employed, preferably at concentrations between 0.05 and 1.0M, and more preferably between 0.1 and 0.5M. A preferred sugar solution is a sucrose solution.

Optionally, the aqueous media may additionally include a proteinase such as Proteinase K to improve the yield of the target nucleic acid. In particular, the inventors have found that including a proteinase with the divalent or trivalent metal ion salt greatly increases the leakage of the target nucleic acid from the cells. The use of Proteinase K and CaCl₂ is particularly preferred.

In a preferred embodiment, the aqueous medium is a low salt buffer. Preferably, the low salt buffer comprises Tris HCl, optionally in combination with EDTA. In this case, preferably the low salt buffer comprises 5mM to 50mM Tris HCl and most preferably about 10mM Tris HCl. Where EDTA is present, it is preferably present at a concentration between 0.1mM and 100mM, more preferably at a concentration between 0.1 and 50mM, and most preferably at a concentration of about 1mM.

In another preferred embodiment, the low salt buffer is a potassium salt at a concentration between 10mM and 30mM, most preferably at a concentration of about 16mM. A preferred pH of potassium salt solutions is about pH4.

Preferably, the aqueous medium has a pH between pH 3 and 11, more preferably between pH 6 and 9, and most preferably a pH of about 8.5.

Slightly higher salt concentrations can also be used, e.g. by employing aqueous media including sodium chloride, e.g. at a concentration which is preferably between about 50mM and 250mM, and more preferably about 150mM. A preferred pH of sodium chloride solutions is about pH7. The salts solutions or aqueous media may be buffered with standard laboratory buffers such as biological buffers, e.g. MES, MOPS, HEPES or Acetate buffers or even phosphate buffers such as PBS.

The aqueous medium may comprise a detergent, either alone or in combination with one or more of the other components described herein. It is preferred that the detergent is a non-ionic detergent such as Tween 20 that do not inhibit subsequent use of the target nucleic acid, e.g. in analytical techniques'. The aqueous medium may further comprise a RNA nuclease or a DNA nuclease to selectively degrade unwanted RNA or DNA targets from a mixture released into the surrounding medium. Additionally, a protease may be employed to degrade any released proteins.

The present invention makes it possible to avoid the harsh conditions used in many prior art methods for purifying nucleic acid. When conditions are made harsher, the cell wall degrades extensively and a considerable amount of unwanted nucleic acids are released. Examples of harsh conditions include, but are not limited to, 0.1M NaOH or 0.1M NaOH with 1% SDS. In some cases, harsh conditions include the use of temperatures of 65°C or above and/or the use of water immiscible organic solvents and/or the use of electroporation. Thus, the method of the invention is carried out in the absence of an electrical field capable of causing poration, e.g. having a field strength above 10,000 V/cm.

In some embodiments, the method may involve one or more of the additional steps of:
(a) isolating the target nucleic acid; or
(b) analysing the target nucleic acid; or
(c) amplifying the target nucleic acid; or
(d) sequencing the target nucleic acid.

These steps are discussed in more detail below.

In a further embodiment of the invention, the target nucleic acid, such as a plasmid, can be separated from the cells according to the present invention and the resulting aqueous media, i.e. the supernatant, used directly with out the requirement for further purification steps, e.g. for PCR or other analytical methods.

A range of techniques are available to the skilled person for purifying nucleic acid that has leaked from cells and is present in the aqueous media. Examples of purification techniques include ion-exchange, electrophoresis, silica solid phase with chaotropic salt extraction, precipitation, flocculation, filtration, gel filtration, centrifugation, alcohol precipitation and/or the use of a charge switch material described in our copending applications European Patent Application No: 98957019.7, United States Patent Application: 09/736,632 and WO 02/48164 and other purification or separation methods well known in the art. In preferred embodiments, the target nucleic acid is purified using a charge switch material, e.g. present on a solid phase, a pipette tip, beads (especially magnetic beads), a porous membrane, a frit, a sinter, a probe or dipstick, a tube (PCR tube, Eppendorf tube) or a microarray. Charge switch materials may be solid phases or soluble. They comprise ionisable groups such that they are capable of binding nucleic acid at a first pH (typically between pH 5.0 and 9.0), and then releasing it at a second higher pH (typically less than 10.5). Examples of solid phase charge switch materials are those in which (1) the ionisable groups are separately immobilised on a solid support by covalent or ionic bonding or by adsorption, (2) the ionisable groups are separately attached to a polymer, said polymer being immobilised on a solid support by covalent or ionic bonding or by adsorption, (3) the ionisable groups are polymerised, optionally by means of cross-linking reagents, and the polymer is immobilised on a solid support by covalent or ionic bonding or by adsorption. By way of example, the charged groups in the charge switch material may be provided by a biological buffer (e.g. Tris, Bis-Tris, polyTris or poly Bis-Tris), a polyhydroxylated amine, a detergent or surfactant, a carbohydrate, a nucleic acid base, a heterocyclic nitrogen-containing compound, a monoamine, a biological dye, or a negatively ionisable group, the pKa of which is between about 3.0 and 7.0 and a metal oxide which is positively charged at said first pH, and optionally also at said second pH.

The target nucleic acid may also be the subject of amplification, conveniently using the polymerase chain reaction. PCR techniques for the amplification of nucleic acid are described in US Patent No: 4,683,195. In general, such techniques require that sequence information from the ends of the target sequence is known to allow suitable forward and reverse oligonucleotide primers to be designed to be identical or similar to the polynucleotide sequence that is the target for the amplification. PCR comprises steps of denaturation of template nucleic acid (if double-stranded), annealing of primer to target, and polymerisation. The nucleic acid probed or used as template in the amplification reaction may be genomic DNA, cDNA or RNA. PCR can be used to amplify specific sequences from genomic DNA, specific RNA sequences and cDNA transcribed from mRNA, bacteriophage or plasmid sequences. References for the general use of PCR techniques include Mullis et al, Cold Spring Harbor Symp. Quant. Biol., 51:263, (1987), Ehrlich (ed), PCR Technology, Stockton Press, NY, 1989, Ehrlich et al, Science, 252:1643-1650, (1991), "PCR protocols; A Guide to Methods and Applications", Eds. Innis et al, Academic Press, New York, (1990).

In some embodiments, the method may involve one or more initial steps such as:
culturing the cells in the culture broth or growing on selective media on plates. The cells/colonies are separated and then placed in the aqueous medium. Separation may be achieved by centrifugation, filtration, magnetic bead separation, or using a probes or dipsticks.

Thus, in one embodiment, the method comprises growing the cells in broth or culture medium, separating the cells and resuspending them in the aqueous media to cause the cells to become leaky and release the target nucleic acid.

Preferred embodiments of the present invention will now be described in more detail, by way of example and not limitation, with reference to the accompanying figures.

### Brief Description of the Figures

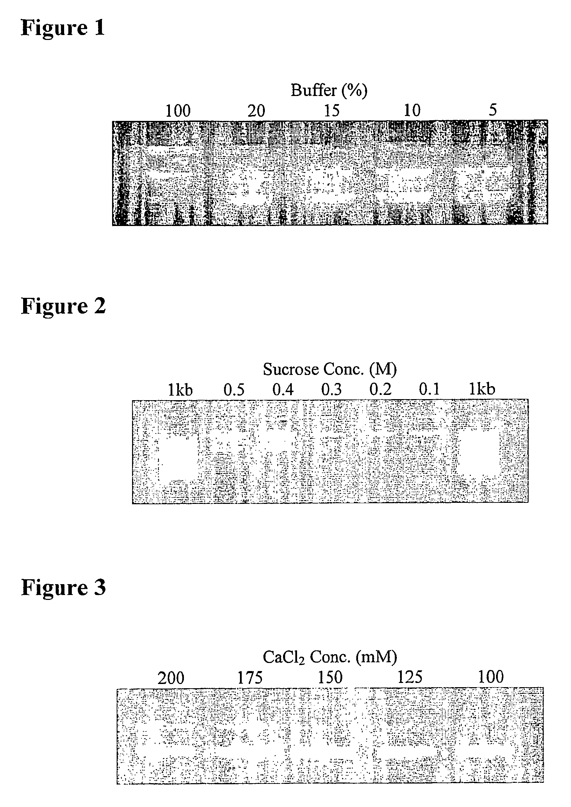

Figures 1 to 5 show the plasmid DNA samples purified according to the present invention run on agarose gels stained with ethidium bromide. The figures are labelled with the conditions (reagents and concentrations) used for each purification. The nucleic acid bands are all from plasmid DNA, in supercoiled, nicked or linear forms. No bands were observed for genomic DNA, demonstrating the selectivity of the method of the invention.

### Example 1:

An overnight 5ml culture of JM109 containing pUC19 plasmid was centrifuged to pellet the cells which were then resuspended in 500µl of 10mM Tris. HCl, 10mM EDTA pH8.5. Following a 5 minute incubation, the cells were centrifuged leaving a clear supernatant containing the plasmid. A sample was taken for PCR analysis and the remaining plasmid was purified by adjusting the pH to 4 with 166µl of a 1.6M potassium acetate buffer and then adding 1mg of magnetic beads derivatised with positively charged groups. The magnetic beads were incubated for 1 minute to bind the plasmid, separated on a magnet, washed twice with water and the DNA recovered by eluting with 100µl of 10mM Tris.HCl pH8.5. Plasmid purity was confirmed by gel electrophoresis and identity confirmed by PCR and sequencing.

### Example 2:

An overnight 5ml culture of XL-1 Blue containing pUC19 plasmid was centrifuged to pellet the cells which were then resuspended in 500µl of 10mM Tris. HCl, 10mM EDTA pH8.5. Following a 5 minute incubation, the cells were centrifuged leaving a clear supernatant containing the plasmid. A sample was taken for PCR analysis and the remaining plasmid was purified by adjusting the pH to 4 with 166µl of a 1.6M potassium acetate buffer and then adding 1mg of magnetic beads derivatised with positively charged groups. The magnetic beads were incubated for 1 minute to bind the plasmid, separated on a magnet, washed twice with water and the DNA recovered by eluting with 100ul of 10mM Tris.HCl pH8.5. Plasmid purity was confirmed by gel electrophoresis and identity confirmed by PCR and sequencing.

### Example 3:

An overnight 5ml culture of XL-1 Blue containing pUC19 plasmid was centrifuged to pellet the cells which were then resuspended in 500µl of water. Following a 5 minute incubation, the cells were centrifuged leaving a clear supernatant containing the plasmid. Plasmid purity was confirmed by gel electrophoresis and identity confirmed by PCR.

### Example 4:

An overnight 5ml culture of XL-1 Blue containing pUC19 plasmid was centrifuged to pellet the cells which were then resuspended in 500µl of 0.15M NaCl. Following a 5 minute incubation, the cells were centrifuged leaving a clear supernatant containing the plasmid. Plasmid purity was confirmed by gel electrophoresis and identity confirmed by PCR.

### Example 5:

An overnight 5ml culture of XL-1 Blue containing pUC19 plasmid was centrifuged to pellet the cells which were then resuspended in 500µl of 10mM potassium acetate, 10mM potassium chloride pH4. Following a 5 minute incubation, the cells were centrifuged leaving a clear supernatant containing the plasmid. Plasmid purity was confirmed by gel electrophoresis and identity confirmed by PCR.

### Example 6:

An overnight 5ml culture of XL-1 Blue containing pUC19 plasmid was centrifuged to pellet the cells which were then resuspended in 500µl of 10mM Tris HCl, 1% Tween 20. Following a 5 minute incubation, the cells were centrifuged leaving a clear supernatant containing the plasmid. Plasmid purity was confirmed by gel electrophoresis and identity confirmed by PCR.

### Example 7:

An overnight 5ml culture of XL-1 Blue containing pUC19 plasmid was centrifuged to pellet the cells which were then resuspended in 500µl of 10mM Tris HCl, 1% Tween 20. Following a 5 minute incubation, the cells and supernatant containing the plasmid were added to a PCR reaction for direct analysis.

### Example 8:

An overnight 5ml culture of JM109 containing pUC19 plasmid was centrifuged to pellet the cells which were then resuspended in 500µl of 10mM Tris. HCl, 10mM EDTA pH8.5 plus RNase A at 20ug/ml. Following a 5 minute incubation, the cells were centrifuged leaving a clear supernatant containing the plasmid. A sample was taken for PCR analysis and the remaining plasmid was purified by adjusting the pH to 4 with 166µl of a 1.6M potassium acetate buffer and then adding 1mg of magnetic beads derivatised with positively charged groups. The magnetic beads were incubated for 1 minute to bind the plasmid, separated on a magnet, washed twice with water and the DNA recovered by eluting with 100µl of 10mM Tris.HCl pH8.5. Plasmid purity was confirmed by gel electrophoresis

### Example 9:

A solution of 10mM Tris HCl, 10mM EDTA, pH8.5 was diluted with water to the following percentage concentrations (%): 100, 20, 15, 10 and 5. Then, 5 x 1.5 ml cultures from 200ml pUC19/XL1-Blue overnight culture were spun down to pellet the cells. The supernatant was removed. About 500µls of the range of buffer concentrations were added to the 5 tubes with 5µl Proteinase K (20mg/ml) and 5µl RNase A (5mg/ml). The 5 samples were fully resuspended and incubated at room temperature for 15 mins. After 15 mins, the samples were spun down again to pellet the cells. The supernatant was collected to a new tube and the pellet was discarded. To each tube, 500µl of a 1.5M potassium acetate buffer pH4 (PB) with 40µl of charge switch magnetic beads were added and the tubes were fully resuspended, incubated for 1 min, separated on a magnetic rack, washed twice and eluted in 50µl Elution Buffer (EB). 10µl of each sample was run on a 1% agarose gel stained with ethidium bromide as shown in Figure 1.

### Example 10:

Sucrose was made up to the following concentrations (M): 0.5, 0.4, 0.3, 0.2 and 0.1. 5 x 1.5 ml cultures from a 200ml pUC19/XL1-Blue overnight culture were spun down to pellet the cells. The supernatant was removed.
500µls of the range of sucrose concentrations (M) were added to the 5 tubes. 5µl Proteinase K and 5µl RNase A was added to each tube as before. The 5 samples were fully resuspended and incubated at room temperature for 15 mins. After 15 mins, the samples were spun down again to pellet the cells. The supernatant was collected to a new tube and the pellet was discarded. 500µl of PB and 40µl of CST magnetic beads were added and the tubes were fully resuspended, incubated for 1 min, separated on a magnetic rack, washed twice and eluted in 50µl Elution Buffer (EB). 10µl of each sample was run on a 1% agarose gel stained with ethidium bromide, as shown in Figure 2.

### Example 11:

CaCl₂ was made up to the following concentrations (mM): 200, 175, 150, 125 and 100. Five x 1.5 ml cultures from a 200µl pUC19/XL1-Blue overnight culture were spun down to pellet the cells. The supernatant was removed. About 500µls of the range of CaCl₂ concentrations were added to the 5 tubes. 5µl Proteinase K and 5µl RNase A was added to each tube as before. The 5 samples were fully resuspended and incubated at room temperature for 15 mins. After 15 mins, the samples were spun down again to pellet the cells. The supernatant was collected to a new tube and the pellet was discarded. 500µl of PB and 40µl of charge switch magnetic beads were added and the tubes were fully resuspended, incubated for 1 min, separated on a magnetic rack, washed twice and eluted in 50µl Elution Buffer (EB). 10µl of each sample was run on a 1% agarose gel stained with ethidium bromide, as show in Figure 3.

### Example 12:

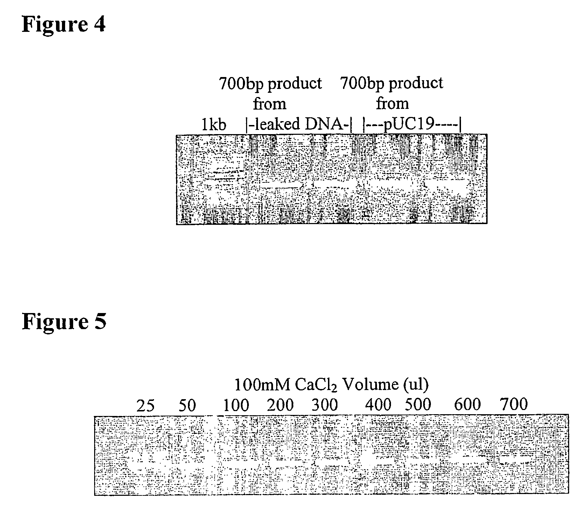

To confirm that the leaked DNA is pUC19 vector plasmid, 4 PCR reactions were set up using pUC19 vector primers to amplify a 700bp PCR fragment. 2 were set up using DNA from a leaky cell extraction and 2 were set up using a sample of pUC19 from laboratory vector stocks. 10µls of each product was run on an ethidium bromide stained 1% agarose gel with a 1kb extension ladder, see Figure 4.

### Example 13:

Nine x 1.5 ml cultures from a 200µl pUC19/XL1-Blue overnight culture were spun down to pellet the cells. The supernatant was removed. The following range of 100mM CaCl₂ volumes (µl) were added to the 9 samples: 25, 50, 100, 200, 300, 400, 500, 600 and 700. 5µl Proteinase K and 5µl RNase A was added to each tube as before. The 9 samples were fully resuspended and incubated at room temperature for 15 mins. After 15 mins, the samples were spun down again to pellet the cells. The supernatant was collected to a new tube and the pellet was discarded. 500µl of precipitation buffer and 40µl of CST magnetic beads were added and the tubes were fully resuspended, incubated for 1 min, separated on a magnetic rack, washed twice and eluted in 50µl Elution Buffer (EB). 10µl of each sample was run on a 1% agarose gel stained with ethidium bromide, see Figure 5.

## Claims

1. A method of obtaining a sample of target nucleic acid from bacterial cells containing the target nucleic acid and genomic nucleic acid, the method comprising the steps of:
separating the cells from culture broth;
suspending the cells in an aqueous medium which causes the target nucleic acid to leak from the cells into the aqueous medium, wherein the aqueous medium is water, a low salt buffer, a salt solution, a divalent or trivalent metal salt solution or a sugar solution; and
obtaining the sample of the nucleic acid from the aqueous medium;
wherein the cells are substantially not lysed during the above steps and substantially retain the genomic nucleic acid within the cells and wherein the method is carried out at a temperature of less than 60°C and in the absence of an electrical field capable of causing cell poration.

2. The method of claim 1, wherein the method is carried out a temperature of less than 40°C.

3. The method of claim 1 or claim 2, wherein cellular proteins are substantially retained within the cells.

4. The method of claim 1, wherein the genomic nucleic acid is host cell chromosomal DNA or ribosomal RNA.

5. The method of any one of the preceding claims, wherein the target nucleic acid sample is a vector, a plasmid, satellite or cosmid DNA or vector RNA.

6. The method of claim 5, wherein the target nucleic acid sample is plasmid DNA.

7. The method of any one of the preceding claims, wherein the cells are a gram negative microorganism.

8. The method of claim 7, wherein the cells are *E. coli.*

9. The method of any one of the preceding claims, wherein the aqueous medium has a pH between pH 6 and 9.

10. The method of any one of the preceding claims, wherein the low salt buffer comprises Tris. HCl.

11. The method of claim 10, wherein the Tris. HCl buffer has a concentration between 5mM and 50mM.

12. The method of claim 10 or claim 11, wherein the Tris. HCl buffer further comprises EDTA.

13. The method of any one of claims 10 to 12, wherein the Tris. HCl buffer has a pH of about 8.5.

14. The method of any one of claims 1 to 9, wherein the low salt buffer comprises potassium acetate/KCl.

15. The method of claim 14, wherein the potassium acetate/KCl has a concentration between 10mM and 30mM.

16. The method of claims 14 or claim 15, wherein the potassium acetate/KCl has a pH of about 4.

17. The method of any one of claims 1 to 9, wherein the salt solution is a sodium chloride solution.

18. The method of claim 17, wherein the sodium chloride solution has a concentration between about 50mM and 250mM and/or a pH of about pH 7.

19. The method of any one of claims 1 to 9, wherein the divalent metal ion solution is a CaCl₂ solution.

20. The method of claim 19, wherein the CaCl₂ solution is at a concentration between 10 and 250mM.

21. The method of any one of claims 1 to 9, wherein the sugar solution is a sucrose solution has a concentration between 0.05 and 1.0M.

22. The method of any one of the preceding claims, wherein the aqueous medium further comprises a proteinase.

23. The method of claim 22, wherein the proteinase is Proteinase K.

24. The method of any one of the preceding claims, wherein the aqueous medium further comprises a detergent.

25. The method of claim 24, wherein the detergent is a non-ionic detergent.

26. The method of any one of the preceding claims, wherein the aqueous medium further comprises a RNA nuclease, and/or a DNA nuclease and/or a protease.

27. A method which comprises the steps of the method of any one of claims 1 to 26 and a further step of purifying the target nucleic acid present in the sample of target nucleic acid.

28. A method which comprises the steps of the method of any one of claims 1 to 26 and a further step of isolating the sample of the target nucleic acid.

29. A method which comprises the steps of the method of any one of claims 1 to 26 and a further step of analysing the sample of the target nucleic acid.

30. A method which comprises the steps of the method of any one of claims 1 to 26 and a further step of amplifying the sample of the target nucleic acid.

31. A method which comprises the steps of the method of any one of claims 1 to 26 and a further step of sequencing the sample of the target nucleic acid.

32. The method of claim 28, wherein the isolation of the target nucleic acid is by ion-exchange, electrophoresis, silica solid phase extraction, precipitation, flocculation, filtration, gel filtration, centrifugation, alcohol precipitation and/or the use of a charge switch material.

## Patentansprüche

1. Verfahren zur Gewinnung einer Target-Nucleinsäure-Probe aus Bakterienzellen, die die Target-Nucleinsäure und genomische Nucleinsäure enthalten, worin das Verfahren die folgenden Schritte umfasst:
Trennen der Zellen vom Kulturmedium;
Suspendieren der Zellen in einem wässrigen Medium, was dazu führt, dass die Target-Nucleinsäure aus den Zellen in das wässrige Medium austritt, worin das wässrige Medium Wasser, ein Puffer mit niedrigem Salzgehalt, eine Salzlösung, eine Lösung eines Salzes eines zweiwertigen oder dreiwertigen Metalls oder eine Zuckerlösung ist; und
Gewinnen der Nucleinsäureprobe aus dem wässrigen Medium;
worin die Zellen während der oben genannten Schritte im Wesentlichen nicht lysiert werden und die genomische Nucleinsäure innerhalb der Zellen im Wesentlichen zurückhalten und worin das Verfahren bei einer Temperatur von weniger als 60 °C und in Abwesenheit eines elektrischen Feldes, das Zellporation verursachen kann, durchgeführt wird.

2. Verfahren nach Anspruch 1, worin das Verfahren bei einer Temperatur von unter 40 °C durchgeführt wird.

3. Verfahren nach Anspruch 1 oder Anspruch 2, worin Zellproteine innerhalb der Zellen im Wesentlichen zurückgehalten werden.

4. Verfahren nach Anspruch 1, worin die genomische Nucleinsäure chromosomale DNA oder ribosomale RNA der Wirtszellen ist.

5. Verfahren nach einem der vorangehenden Ansprüche, worin die Target-Nucleinsäure-Probe ein Vektor, ein Plasmid, Satelliten- oder Cosmid-DNA oder Vektor-RNA ist.

6. Verfahren nach Anspruch 5, worin die Target-Nucleinsäure-Probe Plasmid-DNA ist.

7. Verfahren nach einem der vorangehenden Ansprüche, worin die Zellen ein gramnegativer Mikroorganismus sind.

8. Verfahren nach Anspruch 7, worin die Zellen E. coli sind.

9. Verfahren nach einem der vorangehenden Ansprüche, worin das wässrige Medium einen pH-Wert zwischen pH 6 und 9 aufweist.

10. Verfahren nach einem der vorangehenden Ansprüche, worin der Puffer mit niedrigem Salzgehalt Tris-HCl umfasst.

11. Verfahren nach Anspruch 10, worin der Tris-HCl-Puffer eine Konzentration zwischen 5 mM und 50 mM aufweist.

12. Verfahren nach Anspruch 10 oder Anspruch 11, worin der Tris-HCl-Puffer weiters EDTA umfasst.

13. Verfahren nach einem der Ansprüche 10 bis 12, worin der Tris-HCI-Puffer einen pH von etwa 8,5 aufweist.

14. Verfahren nach einem der Ansprüche 1 bis 9, worin der Puffer mit niedrigem Salzgehalt Kaliumacetat/KCl umfasst.

15. Verfahren nach Anspruch 14, worin das Kaliumacetat/KCl eine Konzentration zwischen 10 mM und 30 mM aufweist.

16. Verfahren nach Anspruch 14 oder Anspruch 15, worin das Kaliumacetat/KCl einen pH von etwa 4 aufweist.

17. Verfahren nach einem der Ansprüche 1 bis 9, worin die Salzlösung eine Natriumchloridlösung ist.

18. Verfahren nach Anspruch 17, worin die Natriumchloridlösung eine Konzentration zwischen etwa 50 mM und 250 mM und/oder einen pH von etwa pH 7 aufweist.

19. Verfahren nach einem der Ansprüche 1 bis 9, worin die Lösung zweiwertiger Metallionen eine CaCl₂-Lösung ist.

20. Verfahren nach Anspruch 19, worin die CaCl₂-Lösung in einer Konzentration zwischen 10 und 250 mM vorliegt.

21. Verfahren nach einem der Ansprüche 1 bis 9, worin die Zuckerlösung eine Saccharoselösung ist und eine Konzentration zwischen 0,05 und 1,0 M aufweist.

22. Verfahren nach einem der vorangehenden Ansprüche, worin das wässrige Medium weiters eine Proteinase umfasst.

23. Verfahren nach Anspruch 22, worin die Proteinase Proteinase K ist.

24. Verfahren nach einem der vorangehenden Ansprüche, worin das wässrige Medium weiters ein Detergens umfasst.

25. Verfahren nach Anspruch 24, worin das Detergens ein nichtionisches Detergens ist.

26. Verfahren nach einem der vorangehenden Ansprüche, worin das wässrige Medium weiters eine RNA-Nuclease und/oder eine DNA-Nuclease und/oder eine Protease umfasst.

27. Verfahren, umfassend die Verfahrensschritte nach einem der Ansprüche 1 bis 26 und einen weiteren Schritt der Reinigung der in der Target-Nucleinsäure-Probe enthaltenen Target-Nucleinsäure.

28. Verfahren, umfassend die Verfahrensschritte nach einem der Ansprüche 1 bis 26 und einen weiteren Schritt des Isolierens der Target-Nucleinsäure-Probe.

29. Verfahren, umfassend die Verfahrensschritte nach einem der Ansprüche 1 bis 26 und einen weiteren Schritt des Analysierens der Target-Nucleinsäure-Probe.

30. Verfahren, umfassend die Verfahrensschritte nach einem der Ansprüche 1 bis 26 und einen weiteren Schritt des Amplifizierens der Target-Nucleinsäure-Probe.

31. Verfahren, umfassend die Verfahrensschritte nach einem der Ansprüche 1 bis 26 und einen weiteren Schritt des Sequenzierens der Target-Nucleinsäure-Probe.

32. Verfahren nach Anspruch 28, worin das Isolieren der Target-Nucleinsäure mittels lonenaustausch, Elektrophorese, Siliciumdioxid-Festphasenextraktion, Fällung, Ausflockung, Filtration, Gelfiltration, Zentrifugation, Alkoholfällung und/oder unter Verwendung eines Ladungswechselmaterials erfolgt.

## Revendications

1. Méthode d'obtention d'un échantillon d'un acide nucléique cible de cellules bactériennes contenant l'acide nucléique cible et un acide nucléique génomique, la méthode comprenant les étapes de:
séparer les cellules du bouillon de culture;
mettre les cellules en suspension dans un milieu aqueux qui force l'acide nucléique cible à fuir des cellules dans le milieu aqueux, où le milieu aqueux est de l'eau, un tampon à faible teneur en sel, une solution de sel, une solution d'un sel d'un métal divalent ou trivalent ou une solution de sucre; et
obtenir l'échantillon de l'acide nucléique du milieu aqueux;
où les cellules ne sont sensiblement pas lysées pendant les étapes ci-dessus et conservent sensiblement l'acide nucléique génomique dans les cellules et où la méthode est effectuée à une température de moins 60°C et en l'absence d'un champ électrique capable de provoquer une poration des cellules.

2. Méthode de la revendication 1, où la méthode est effectuée à une température de moins de 40°C.

3. Méthode de la revendication 1 ou de la revendication 2, où les protéines cellulaires sont sensiblement retenues dans les cellules.

4. Méthode de la revendication 1, où l'acide nucléique génomique est l'ADN chromosomique ou l'ADN ribosomique de la cellule hôte.

5. Méthode de l'une quelconque des revendications précédentes, où l'échantillon d'acide nucléique cible est un vecteur, un plasmide, un satellite ou un ADN cosmidique ou un ARN vectoriel.

6. Méthode de la revendication 5, où l'échantillon d'acide nucléique cible est un ADN plasmidique.

7. Méthode de la revendication précédente, où les cellules sont un microorganisme gram négatif.

8. Méthode de la revendication 7, où les cellules sont *E. coli.*

9. Méthode de l'une quelconque des revendications précédentes, où le milieu aqueux a un pH compris entre pH 6 et 9.

10. Méthode de l'une quelconque des revendications précédentes, où le tampon à faible teneur en sel comprend Tris.HCl.

11. Méthode de la revendication 10, où le tampon Tris. HCl a une concentration comprise entre 5mM et 50mM.

12. Méthode de la revendication 10 ou de la revendication 11, où le tampon Tris. HCl comprend de plus EDTA.

13. Méthode de l'une quelconque des revendications 10 à 12, où le tampon Tris. HCl a un pH d'environ 8,5.

14. Méthode de l'une quelconque des revendications 1 à 9, où le tampon à faible teneur en sel comprend de l'acétate de potassium/KCl.

15. Méthode de la revendication 14, où l'acétate de potassium/KCl a une concentration comprise entre 10 mM et 30mM.

16. Méthode de la revendication 14 ou de la revendication 15, où l'acétate de potassium/KCl a un pH d'environ 4.

17. Méthode de l'une quelconque des revendications 1 à 9, où la solution de sel est une solution de chlorure de sodium.

18. Méthode de la revendication 17, où la solution de chlorure de sodium a une concentration comprise entre environ 50mM et 250mM et/ou un pH d'environ pH 7.

19. Méthode de l'une quelconque des revendications 1 à 9, où la solution de l'ion d'un métal divalent est une solution de CaCl₂.

20. Méthode de la revendication 19, où la solution de CaCl₂ est à une concentration comprise entre 10 et 250mM.

21. Méthode de l'une quelconque des revendications 1 à 9, où la solution de sucre est une solution de saccharose à une concentration comprise entre 0,05 et 1,0M.

22. Méthode de l'une quelconque des revendications précédentes, où le milieu aqueux comprend de plus une protéinase.

23. Méthode de la revendication 22, où la protéinase est la Protéinase K.

24. Méthode de l'une quelconque des revendications précédentes, où le milieu aqueux comprend de plus un détergent.

25. Méthode de la revendication 24, où le détergent est un détergent non ionique.

26. Méthode de l'une quelconque des revendications précédentes, où le milieu aqueux comprend de plus une ARN nucléase, et/ou une ADN nucléase et/ou une protéase.

27. Méthode qui comprend les étapes de la méthode de l'une quelconque des revendications 1 à 26 et une autre étape de purification de l'acide nucléique cible présent dans l'échantillon de l'acide nucléique cible.

28. Méthode qui comprend les étapes de la méthode de l'une quelconque des revendications 1 à 26 et une autre étape d'isolement de l'échantillon de l'acide nucléique cible.

29. Méthode qui comprend les étapes de la méthode de l'une quelconque des revendications 1 à 26 et une autre étape d'analyse de l'échantillon de l'acide nucléique cible.

30. Méthode qui comprend les étapes de la méthode de l'une quelconque des revendications 1 à 26 et une autre étape d'amplification de l'échantillon de l'acide nucléique cible.

31. Méthode qui comprend les étapes de la méthode de l'une quelconque des revendications 1 à 26 et une autre étape de séquençage d'un échantillon de l'acide nucléique cible.

32. Méthode de la revendication 28, où l'isolement de l'acide nucléique cible est par échange d'ions, et électrophorèse, extraction en phase solide de silice, précipitation, flocculation, filtration, filtration sur gel, centrifugation, précipitation à l'alcool et/ou utilisation d'une matière de changement de charge.
